# DEMANDE DE BREVET EUROPEEN

(11) **EP 4 537 869 A1**
(43) Date de publication de la demande: **16.04.2025**
(21) Numéro de dépôt: 23202973.6
(22) Date de dépôt: 11.10.2023
(51) Int. Cl.: A61M 5/00, B01L 3/00, B01L 9/06, B65B 35/00

(54) **DISPOSITIF DE RENESTAGE DE SERINGUES DE SUBSTANCE PHARMACEUTIQUE OU COSMÉTIQUE**

(71) Demandeur: Cilyx, 4102 Seraing (BE)
(72) Inventeur: MARON, Olivier, 4102 Seraing (BE)
(74) Mandataire: Calysta NV

(57) **Abrégé**

Dispositif et procédé de remise en nest (8) de seringues (2) à partir d'un convoyeur de transport (15) comprenant un châssis (9) comprenant une entrée (13) agencée pour permettre le passage de seringues (2), un bloc peigne (25) muni de dents de séparation (29) juxtaposées et d'espaces interdentaires (30), un peigne mobile (31) muni de dents de séparation (37) et d'espaces interdentaires (38), ledit peigne mobile (31) étant agencé pour coulisser transversalement par rapport à ladite direction d'alimentation (A) dans la direction du peigne bloc (25) et loger les seringues (2) en matrice.

## Description

La présente invention se rapporte à un dispositif de renestage de seringues de substance pharmaceutique ou cosmétique.

Les substances pharmaceutique ou cosmétique sont typiquement conditionnées en seringues. Ceux-ci sont livrés en nest aux acteurs de l'industrie pharmaceutique ou cosmétique, dans un boîtier (Tub) de protection qui protège les conteneurs de la casse, les conteneurs étant disposés parallèlement et maintenus substantiellement écartés par le nest.

Les nests sont des présentoirs pour seringues et sont munis d'une plaque basale munie de moyens de retenue dans lesquels les conteneurs sont enfoncés. Au moment de la livraison, dans le nest se trouvent les corps de conteneur à remplir de la substance pharmaceutique ou cosmétique. Le corps de seringue comporte une collerette.

Ensuite, la seringue est munie de son aiguille avec un bouchon et une fois remplie, la seringue sera ensuite pourvue d'un bouchon et d'un piston.

De la livraison des corps de seringues jusqu'au conditionnement final des seringues, après remplissage, de nombreuses manipulations de ces seringues sont réalisées. Ces manipulations sont réalisées généralement par des bras robotisés munis de pinces ou de préhenseurs qui fonctionnent de différentes manières. Les pinces/préhenseurs conventionnels fonctionnent pneumatiquement et sont pourvues de petites ventouses qui permettent la prise des seringues. Ces bras robotisés permettent de fonctionner à haute cadence, typiquement ils permettent la manipulation de plus de 300 conteneurs/min, parfois 600 conteneurs/min.

Un enjeu fréquent de la manipulation des seringues est la modification du positionnement en matrice dans le nest au convoyage en ligne ou inversement. Cet enjeu se présente au moment de la livraison des corps de seringues vides ou au moment de la manipulation de seringues pré-remplies, munies typiquement de leurs bouchons, mais pas nécessairement. Typiquement, lorsque les seringues sont pré-remplies dans une ligne de remplissage, elles ne sont pas encore munies de leur piston.

C'est d'ailleurs dans ce contexte que les pinces robotisées permettent d'enlever une rangée de seringues du nest et placent les conteneurs en ligne, les uns derrières les autres sur le convoyeur.

Si les bras robotisés sont aujourd'hui très bien développés et fréquemment utilisés, ceux-ci ne trouvent une réelle utilité que lorsque la cadence est importante. En effet, dans le cas de production de petites séries ou de petits lots, ou encore dans le cas de convoyeurs plus lents comme ceux qui permettent l'inspection des conteneurs avant utilisation ou placement du piston, par une mireuse surveillée par un opérateur, l'utilisation d'un bras robotisé n'est pas justifiée et le coût de l'automatisation n'est pas absorbé sur la taille de la série. Parfois aussi, la cadence requise ne permet pas l'utilisation d'un bras robotisé, celui-ci n'étant pas prévu pour travailler à faible cadence.

Dans ce contexte, il existe des pinces manuelles que l'opérateur prend en main, qui lui permettent de réaliser la préhension de seringues par rangée dans le nest et qu'il positionne ensuite en entrée de convoyeur afin d'y déposer ces seringues.

Malheureusement, cette étape doit être répétée un grand nombre de fois sur la taille d'une petite série, ce qui crée une grande pénibilité du travail, demande un temps considérable pour le renestage (remise en nest des conteneurs) ou le dénestage (sortie des conteneurs du nest) ainsi que des risques de casse.

L'invention a pour but de pallier les inconvénients de l'état de la technique en procurant un dispositif de renestage de seringues qui permet de réduire la pénibilité de l'opération de renestage manuelle, d'augmenter la vitesse ainsi que de limiter le risque de casse.

Pour résoudre ce problème, il est prévu suivant l'invention un dispositif de remise en nest d'au moins n sur x (n multiplié par x) seringues de substance pharmaceutique ou cosmétique (renestage) tel qu'indiqué au début comprenant
- un châssis sensiblement parallélépipédique comprenant une partie dorsale agencée pour être reliée à un convoyeur de transport de seringues de substance pharmaceutique ou cosmétique selon une direction d'alimentation en au moins une file, ladite partie dorsale s'étendant vers le haut à partir d'une base du châssis et comprenant une entrée agencée pour permettre le passage de seringues en provenance du convoyeur de transport dans le châssis, une partie frontale, s'étendant également vers le haut à partir de ladite base du châssis et faisant face à ladite partie dorsale, la partie dorsale et la partie frontale étant munis de moyens de coulissement disposé à une hauteur h1 mesurée à partir de ladite base dudit châssis,
- un bloc peigne muni d'une première partie latérale et d'une deuxième partie latérale s'étendant entre une base de bloc et un ciel de bloc peigne, et comprenant, entre ladite première partie latérale et ladite deuxième partie latérale, n -1 dents de séparation juxtaposées, et n espaces interdentaires, les dites dents de séparation et lesdits espaces interdentaires étant orientés de manière sensiblement transversale par rapport à la direction d'alimentation, et disposé de telle façon que le ciel du bloc peigne soit disposé à une hauteur h₂ mesurée à partir de la base dudit châssis, chacun des n espaces interdentaires étant agencé pour accueillir x seringues,
- un peigne mobile muni d'un ciel de peigne mobile et d'une base de peigne mobile de laquelle s'étendent une première partie latérale et une deuxième partie latérale et d'une poignée, comprenant, entre ladite première partie latérale et ladite deuxième partie latérale, x-1 dents de séparation et x espaces interdentaires, lesdites dents de séparation et lesdits espaces interdentaires étant orientés de manière sensiblement parallèle à la direction d'alimentation et comprenant des moyens de coulissement mutuel aux moyens de coulissements de la partie dorsale et de la partie frontale dudit châssis, ledit peigne mobile présentant une position de déplacement où il est agencé pour coulisser en appui sur ladite partie dorsale et ladite partie frontale dudit châssis et se déplacer transversalement par rapport à ladite direction d'alimentation dans la direction du peigne bloc et où ladite base de peigne mobile est positionnée à une hauteur h₃ mesurée à partir de la base dudit châssis supérieure à h₂, lesdits espaces interdentaires étant agencés pour loger n seringues provenant dudit convoyeur de transport.

Comme on peut le constater, selon la présente invention, le dispositif de renestage comprend un châssis qui est placé en bout de convoyeur en ligne dans lequel une partie dorsale comprend une entrée au travers de laquelle passent les seringues issues du convoyeur.

Le dispositif de renestage comprend également un bloc peigne qui comprend une série de n-1 dents de séparation entre deux parois latérales situées de part et d'autre des dents de séparation qui forment ensemble n espaces interdentaires. Ces n espaces interdentaires sont prévus pour accueillir chacun une série de x seringues, présentant ainsi chacun une longueur suffisante pour accueillir ladite série. En d'autres mots, la longueur de chaque espace interdentaire est supérieure au diamètre des seringues multiplié par le nombre de seringues, soit x.

Les dents de séparation du bloc peigne permettent ainsi de séparer les séries de x seringues contenues dans les espaces interdentaires les unes des autres de telle façon que la distance qui sépare les séries de x seringues logées dans les espaces interdentaires soit sensiblement la même que celle qui sépare les séries de x seringues logées dans un nest.

Le dispositif de renestage comprend par ailleurs un peigne mobile qui comprend une série de x-1 dents de séparation entre deux parties latérales situées de part et d'autre des dents de séparation qui forment ensemble x espaces interdentaires. Ces x espaces interdentaires sont prévus pour accueillir chacun une série de n seringues, présentant ainsi chacun une longueur suffisante pour accueillir ladite série. En d'autres mots, la longueur de chaque espace interdentaire est supérieure au diamètre des seringues multiplié par le nombre de seringues, soit n.

Le peigne mobile comporte des moyens de coulissement mutuels à ceux qui équipent le châssis. Les moyens de coulissement mutuels du peigne mobile sont engagés dans ceux du châssis, présents sur la partie dorsale et permettent un déplacement du peigne mobile perpendiculairement à la direction d'alimentation de seringues. Le peigne mobile peut ainsi se déplacer selon au moins x déplacements consécutifs. La position de déplacement du peigne mobile est celle où les moyens de coulissement mutuels du peigne mobile sont engagés dans les moyens de coulissement du châssis. Lors du premier déplacement du peigne mobile, le premier espace interdentaire fait face à l'ouverture de la partie dorsale pour accueillir n seringues dans celui-ci. Ensuite, il est déplacé en direction du peigne bloc et la deuxième espace interdentaire fait face à l'ouverture de la partie dorsale pour également accueillir n seringues dans celui-ci. Cette opération est reproduite jusqu'à avoir le xième espace interdentaire qui fait face à l'ouverture de la partie dorsale pour également accueillir n seringues dans celui-ci. Une fois le dernier (xième) espace interdentaire rempli des n seringues, le peigne mobile est encore déplacé en direction dudit peigne bloc.

Le peigne mobile comporte une base de et un ciel de peigne mobile. La base du peigne mobile est positionnée à une hauteur mesurée à partir de la base dudit châssis h₃ supérieure à h₂. La hauteur h₂ étant la hauteur à laquelle culmine le ciel du bloc peigne. De cette façon, lorsque le peigne mobile se déplace en coulissant en direction du peigne bloc, il recouvre le peigne bloc et les n seringues logées dans chacun des espaces interdentaires du peigne mobile sont engagées dans les n espaces interdentaires du bloc peigne. Ainsi les x rangées de n seringues logées dans les x espaces interdentaires du peigne mobile sont logées dans les n colonnes du bloc peigne, au fur et à mesure du déplacement du peigne mobile jusqu'à ce que chaque espace interdentaire du bloc peigne contienne une série de x seringues.

De cette manière, en utilisant le dispositif selon la présente invention, l'opérateur peut alors saisir le peigne mobile qui repose sur les moyens de coulissement dudit châssis dans lequel les seringues pendent entre les dents, dans les espaces interdentaires selon une matrice de n seringues sur x seringues correspondant aux orifices du nest de n sur x seringues, pour effectuer la remise en nest en le soulevant et en introduisant les seringues dans le nest.

Par les termes « seringues », on entend selon la présente invention un corps de seringue, plus particulièrement en verre, munie ou à munir d'une aiguille et d'un capuchon muni en partie supérieure d'une collerette ou un tube pour injecteur sans aiguille pour autant qu'il soit muni en partie supérieure d'une collerette.

Avantageusement, dans le dispositif de renestage ou remise en nest d'au moins n sur x seringues de substance pharmaceutique ou cosmétique selon la présente invention, ledit peigne mobile est agencé pour se déplacer selon un déplacement transversal par rapport à ladite direction d'alimentation entre une première position p₁ et une dernière position p, en passant par x position successives, p₂, p₃, ...pₓ, ladite première position étant une position où le premier des x espaces interdentaire fait face à ladite entrée de la partie dorsale dudit châssis, ladite dernière position p étant une position où chacun des x espaces interdentaire a fait face à ladite entrée de ladite partie dorsale dudit châssis et où les espaces interdentaires x contiennent les seringues de substance pharmaceutique ou cosmétique, ledit peigne mobile étant alors positionné dans ladite dernière position de telle manière qu'il recouvre au moins partiellement ledit bloc peigne.

Dans une forme de réalisation préférée de la présente invention, ledit peigne mobile comprend une poignée, disposée au-dessus du ciel de peigne mobile, reliée par des moyens de connexion à l'un des éléments parmi le ciel de peigne mobile, ladite première partie latérale et ladite deuxième partie latérale ou lesdits moyens de coulissement mutuels.

Dans une forme de réalisation particulière de la présente invention, ledit peigne mobile comprend des espaces interdentaires pairs agencés pour loger chacun une série de n seringues et former des rangées paires de n seringues et des espaces interdentaires impairs agencés pour loger chacun une série de n seringues et former des rangées impaires de n seringues, et comprend des moyens de centrage agencés pour décaler les rangées paires de n seringues des rangées impaires de n seringues.

Plus particulièrement, selon la présente invention, les moyens de centrage comprennent
- une première plaque dans laquelle sont présents des orifices longitudinaux orientés dans une direction parallèle aux dents du peigne mobile, lesdits orifices longitudinaux surplombant les espaces interdentaires pairs, ladite première plaque étant positionnée au-dessus des dents du peigne mobile lorsque celui-ci est en position de déplacement et,
- une deuxième plaque positionnée au-dessus de ladite première plaque lorsque le peigne mobile est en position de déplacement et comprenant des saillies longitudinales selon une direction parallèle aux dents du peigne mobile, lesdites saillies longitudinales étant agencées pour passer au travers desdits orifices longitudinaux de la première plaque et de longueur inférieure à la longueur desdits orifices longitudinaux et comprenant sous lesdites saillies, des éléments de reliefs, plus particulièrement n éléments de reliefs s'étendant de ladite deuxième plaque vers le bas, lesdits éléments de relief surplombant les espaces interdentaires impairs et étant agencés pour engager les orifices des corps des seringues présentes dans les espaces interdentaires impairs,
- des moyens de commande permettant un déplacement de ladite deuxième plaque par rapport à ladite première plaque lors duquel lesdites saillies longitudinales de la deuxième plaque effectuent un déplacement longitudinal dans les orifices longitudinaux de la première plaque, ledit déplacement longitudinal permettant de déplacer les n seringues présentes dans les espaces interdentaires pairs par rapport aux n seringues présentes dans les espaces interdentaires impairs dudit peigne mobile.

Dans une variante particulière, selon la présente invention, les moyens de centrage comprennent :
- une première plaque dans laquelle sont présents des orifices longitudinaux orientés dans une direction parallèle aux dents du peigne mobile, lesdits orifices longitudinaux surplombant les espaces interdentaires impairs, ladite première plaque étant positionnée au-dessus des dents du peigne mobile lorsque celui-ci est en position de déplacement et,
- une deuxième plaque positionnée au-dessus de ladite première plaque lorsque le peigne mobile est en position de déplacement et comprenant des saillies longitudinales selon une direction parallèle aux dents du peigne mobile, lesdites saillies longitudinales étant agencées pour passer au travers desdits orifices longitudinaux de la première plaque et de longueur inférieure à la longueur desdits orifices longitudinaux et comprenant sous lesdites saillies, des éléments de reliefs plus particulièrement n éléments de reliefs s'étendant de ladite deuxième plaque vers le bas, lesdits éléments de relief surplombant les espaces interdentaires pairs et étant agencés pour engager les orifices des corps des seringues présentes dans les espaces interdentaires pairs,
- des moyens de commande permettant un déplacement de ladite deuxième plaque par rapport à ladite première plaque lors duquel lesdites saillies longitudinales de la deuxième plaque effectuent un déplacement longitudinal dans les orifices longitudinaux de la première plaque, ledit déplacement longitudinal permettant de déplacer les n seringues présentes dans les espaces interdentaires impairs par rapport aux n seringues présentes dans les espaces interdentaires pairs dudit peigne mobile.

Dans un autre mode de réalisation selon la présente invention, ladite entrée de la partie dorsale est une ouverture en forme de U, dont l'ouverture est vers le haut, et d'une largeur l₁, formée dans une paroi de la partie dorsale, dont les côtés forment à proximité de ladite ouverture une zone d'appui pour permettre à des collerettes de seringues de reposer, ladite largeur l₁ étant supérieure au diamètre de corps des seringues.

Plus particulièrement, selon la présente invention, ladite partie dorsale du châssis comprend une paroi transversale qui s'étend de la partie dorsale, de préférence de la paroi de la partie dorsale lorsqu'elle est présente, vers la partie frontale, ledit moyen de coulissement dont est munie la partie dorsale étant disposé sur une face supérieure de la paroi transversale.

Dans une forme de réalisation particulièrement avantageuse de la présente invention, une paroi supérieure est positionnée au-dessus de la paroi de la partie dorsale, ladite paroi supérieure comportant une ouverture en forme de U, dont l'ouverture est orientée vers le bas, et présentant une largeur l₂ supérieure à la largeur l₁, l'ouverture de la paroi de la partie dorsale et l'ouverture de la paroi supérieure formant ensemble une ouverture en forme de T, dont les épaulements forment ladite zone d'appui pour permettre à des collerettes de seringues de reposer.

Dans une autre forme de réalisation particulièrement avantageuse de la présente invention, ladite entrée de la partie dorsale est munie de moyens de fermeture, agencés pour entraver le passage des seringues.

Dans une forme de réalisation avantageuse selon la présente invention, la partie dorsale comprend un rail de guidage muni de creux et le peigne mobile comprend un système de billes sur ressort, lesdits creux étant agencées pour recevoir lesdites billes sur ressort dudit peigne mobile. De cette façon, au fur et à mesure de l'avancement du peigne mobile dans les moyens de coulissement, la bille sur ressort est logée dans des creux consécutifs et le peigne mobile est ainsi temporairement retenu dans une position dans laquelle un espace interdentaire du peigne mobile est positionné face à ladite entrée de la partie dorsale.

En d'autres termes, quand la bille sur ressort du peigne mobile est logée dans un creux dans le rail de guidage de la partie dorsale, le peigne mobile est positionné de telle manière qu'un espace interdentaire du peigne mobile est positionné face à ladite entrée de la partie dorsale. Quand le peigne mobile avance, la bille sur ressort est logée dans un creux suivant dans le rail de guidage de la partie dorsale et le peigne mobile est positionné de telle manière qu'un espace interdentaire suivant est positionné face à ladite entrée de la partie dorsale et ainsi de suite.

Dans encore une autre forme de réalisation particulière de la présente invention, ladite partie dorsale comprend une came crantée agencée pour recevoir ledit peigne mobile en appui et pour permettre son déplacement en position de déplacement de pas en pas, chaque pas correspondant à une position où un espace interdentaire du peigne mobile est positionné face à ladite entrée de la partie dorsale.

De manière avantageuse, selon la présente invention, les dents dudit peigne mobile présentent chacune une extrémité libre et une extrémité liée entre lesquelles s'étend un corps de dent, chaque extrémité libre des dents présentant une largeur s'amenuisant du corps de dent vers l'arête distale de ladite extrémité libre.

Plus particulièrement, selon la présente invention, les dents dudit bloc peigne présentent chacune une extrémité libre et une extrémité liée entre lesquelles s'étend un corps de dent, chaque extrémité libre des dents présentant une largeur s'amenuisant du corps de dent vers l'arête distale de ladite extrémité libre.

De manière particulièrement préférée, selon l'invention, chaque corps de dent dudit bloc peigne présente une largeur au niveau de l'extrémité libre inférieure à une largeur au niveau de l'extrémité liée, de manière à permettre d'écarter les seringues les unes des autres.

Dans un mode particulier de la présente invention, une butée est présente, s'étendant de la base dudit châssis vers le haut, et agencée pour se déplacer dans la direction d'alimentation des seringues, en face de ladite ouverture de ladite partie dorsale , ladite butée étant agencée pour retenir les seringues en provenance de ladite ouverture, avant que ledit peigne mobile présente le premier espace interdentaire et après que le peigne mobile présente le dernier espace interdentaire à ladite ouverture de la partie dorsale.

En effet, selon la présente invention, avant la présentation par le peigne mobile du premier espace interdentaire en face de l'ouverture dans la partie dorsale, le peigne mobile ouvre et verrouille la butée en position ouverte. Lorsque le peigne mobile a présenté tous les espaces interdentaires à remplir de seringues à l'ouverture dans la partie dorsale, le peigne déverrouille la butée pour qu'elle passe de la position ouverte à la position fermée, position dans laquelle les seringues en provenance de l'ouverture de la partie dorsale sont retenues par ladite butée.

D'autres formes de réalisation du dispositif de renestage ou de remise en nest d'au moins n sur x seringues de substance pharmaceutique ou cosmétique suivant l'invention sont indiquées dans les revendications annexées.

L'invention a aussi pour objet un procédé de remise en nest d'au moins n sur x seringues de substance pharmaceutique ou cosmétique en provenance d'un convoyeur de transport auquel est fixé un châssis muni d'un bloc peigne comprenant une première partie latérale et une deuxième partie latérale s'étendant entre une base de bloc et un ciel de bloc peigne, et comprenant, entre ladite première partie latérale et ladite deuxième partie latérale, n -1 dents de séparation juxtaposées, et n espaces interdentaires et d'un peigne mobile muni d'un ciel de peigne mobile et d'une base de peigne mobile de laquelle s'étendent une première partie latérale et une deuxième partie latérale et d'une poignée, ledit peigne mobile comprenant, entre ladite première partie latérale et ladite deuxième partie latérale, x-1 dents de séparation et x espaces interdentaires, comprenant :
- Une alimentation de seringues selon une direction d'alimentation en ligne dans un premier espace interdentaire dudit peigne mobile, lors de laquelle la première partie latérale dudit peigne mobile est positionné au-dessus dudit ciel de bloc peigne, jusqu'à obtenir une première rangée de n seringues dans ledit premier espace interdentaire x₁ dudit peigne mobile,
- Une série de x-1 cycle comprenant chacun un déplacement du peigne mobile selon une direction perpendiculaire à ladite direction d'alimentation en direction dudit bloc peigne, chaque déplacement permettant d'aligner un xième espace interdentaire en face de ladite ouverture et d'engager chacune des n seringues d'une rangée précédentes de n seringues dans un espace interdentaire des n espaces interdentaires dudit bloc peigne, et une alimentation de n seringues dans le xième espace interdentaire jusqu'à obtenir une xième rangée de n seringue dans ledit xième espace interdentaire dudit peigne mobile,

- Un blocage de la position des seringues de x séries de n seringues logées dans le peigne mobile et écartées les unes des autres par ledit bloc peigne pour former une position de transfert desdites seringues logées dans ledit peigne mobile,
- Un levage dudit peigne mobile de manière à retirer lesdites seringues bloquées en position de transfert dudit bloc peigne,
- Un déplacement dudit peigne mobile jusqu'à le positionner au-dessus d'un nest à seringues de n sur x, de telle manière que lesdites seringues logées dans ledit peigne mobile soient placées au-dessus des orifices correspondants dans un nest à seringues,
- Un engagement partiel desdites seringues dans lesdits orifices correspondant dudit nest,
- Un retrait dudit peigne mobile par déplacement sensiblement horizontal, permettant de libérer lesdites seringues logées dans ledit peigne mobile et partiellement dans lesdits orifices du nest et leur descente dans l'orifice correspondant une fois libérée dudit peigne mobile pour obtenir une mise en nest des seringues

Dans une forme de réalisation avantageuse, le procédé selon la présente invention comprend préalablement audit blocage de la position des seringues logées dans le peigne mobile, un déplacement des seringues des rangées paires par rapport aux seringues des rangées impaires de manière à former des rangées de seringues en quinconce les unes par rapport aux autres.

D'autres formes de réalisation du procédé suivant l'invention sont indiquées dans les revendications annexées. D'autres caractéristiques, détails et avantages de l'invention ressortiront de la description donnée ci-après, à titre non limitatif et en faisant référence aux dessins.

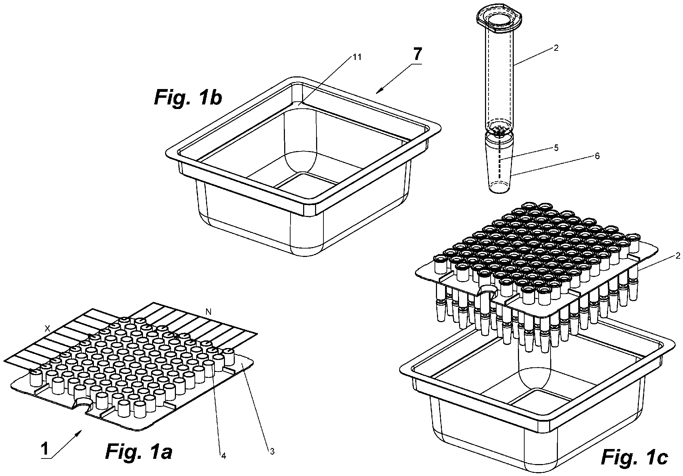

Dans les dessins, la figure 1a est une vue en perspective d'un nest à seringues, la figure 1b est une vue en perspective d'un tub ou boîtier destiné à contenir un nest tandis que la figure 1c est une vue éclatée d'un nest contenant des seringues dans un tub ou boîtier.

La figure 2 est une vue en perspective illustrant le châssis et le bloc peigne du dispositif selon la présente invention.

La figure 3 est une vue en perspective d'une forme de réalisation du peigne mobile selon la présente invention.

La figure 4 est une vue en perspective d'une autre forme de réalisation du peigne mobile selon la présente invention

La figure 5 est une vue frontale du peigne mobile selon la présente invention

La figure 6 est une vue en perspective du dispositif de renestage selon la présente invention, comprenant le châssis et le bloc peigne muni du peigne mobile en position de déplacement.

Les figures 7a et 7 b sont des vues de dessous du peigne mobile selon la présente invention dans lesquelles respectivement le dispositif de centrage est inactif et le dispositif de centrage est activé.

Sur les figures, les éléments identiques ou analogues portent les mêmes références

Comme on peut le voir à la figure 1a, un nest 1, comme par exemple, un nest 1 à seringues 2 est un présentoir muni d'une plaque basale 3 de laquelle s'étendent des moyens de retenue 4 dans lesquels les seringues 2 sont introduites comme illustré à la figure 1c, munies de leurs aiguilles 5 avec un bouchon 6. Les nests 1 sont typiquement placés dans des boîtiers ou tub 7 (voir figure 1b) qui comprend un épaulement intérieur 11 sur son pourtour sur lequel repose la plaque basale 3 du nest 1.

Le nest 1 une fois rempli comprend x séries de n seringues 2 disposées en matrice ou n séries de x seringues 2. On appelle ainsi le nest un nest de x sur n seringues.

La présente invention concerne un dispositif de renestage de seringues également appelé un dispositif de remise en nest d'au moins n sur x (n multiplié par x) seringues permettant de réduire la pénibilité de l'opération de renestage manuelle, d'augmenter la vitesse ainsi que de limiter le risque de casse.

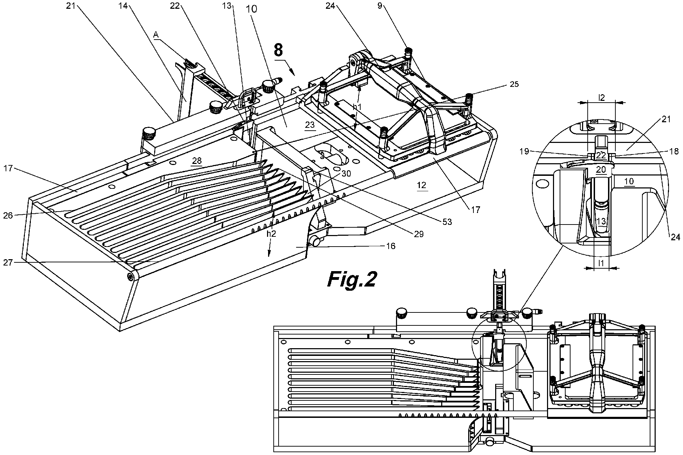

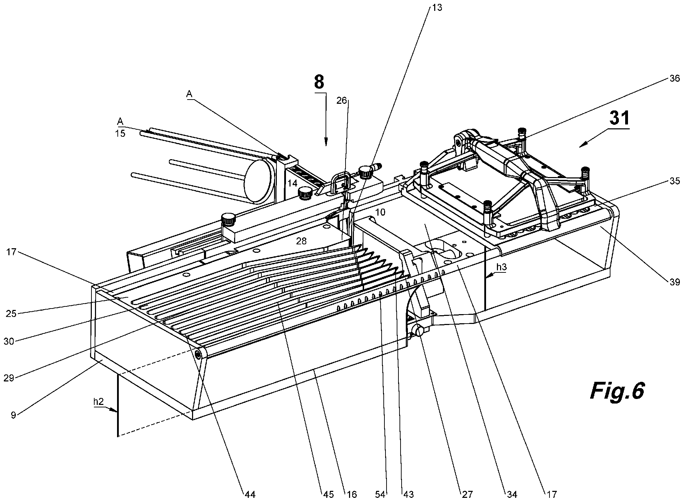

Le dispositif de renestage 8 selon la présente invention est illustré en perspective aux figure 2 et 6. Comme on peut le voir en détail à la figure 2, le dispositif de renestage 8 comprend un châssis 9 sensiblement parallélépipédique comprenant une partie dorsale 10 agencée pour être reliée à un convoyeur de transport de seringues selon une direction d'alimentation A en au moins une file de seringue ou ligne de seringues. Ladite partie dorsale 10 s'étendant vers le haut à partir d'une base 12 du châssis et comprenant une entrée 13 agencée pour permettre le passage de seringues en provenance du convoyeur de transport 15 (illustré à la figure 6) dans le châssis 9, éventuellement via un moyen de guidage 14, une partie frontale 16, s'étendant également vers le haut à partir de ladite base 12 du châssis 9 et faisant face à ladite partie dorsale 10.

La partie dorsale et la partie frontale étant munis de moyens de coulissement 17 disposé à une hauteur h1 mesurée à partir de ladite base dudit châssis 9. Le châssis 9 illustré à la figure 2 est un caisson dont le fond 12 et les parois 10 et 16 sont sensiblement pleines et où l'entrée de la partie dorsale est une ouverture en forme de U, dont l'ouverture est vers le haut, et d'une largeur l₁, formée dans une paroi de la partie dorsale 10, dont les côtés supérieurs 18; 19 forment à proximité de ladite ouverture une zone d'appui pour permettre à des collerettes de seringues de reposer, ladite largeur l₁ étant supérieure au diamètre de corps des seringues. L'entrée 13 de la partie dorsale 10 est munie de moyens de fermeture 20, agencés pour entraver le passage des seringues. Les moyens de fermetures 20 sont commandables, soit manuellement, soit reliés à un dispositif de commande à capteurs qui actionne les moyens de fermeture 20 lorsque n seringues sont passées par l'entrée 13.

Lorsque les moyens de fermeture sont commandés manuellement, une extrémité du peigne mobile 31 vient engager l'extrémité libre du moyen de fermeture et le fait aussi basculer vers le haut, ce qui libère l'entrée 13 du châssis pour permettre le passage des seringues.

Une paroi supérieure 21 est positionnée au-dessus de la paroi de la partie dorsale 10 et comporte une ouverture 22 en forme de U, dont l'ouverture est orientée vers le bas, et présentant une largeur l₂ supérieure à la largeur l₁. L'ouverture de la paroi de la partie dorsale et l'ouverture de la paroi supérieure forment ensemble une ouverture en forme de T, dont les épaulements forment ladite zone d'appui pour permettre à des collerettes de seringues de reposer.

Les moyens de coulissement 17 de la partie dorsale 10 sont positionnés sur une paroi additionnelle 23, sur le bord supérieur 24 de celle-ci.

Alternativement, la partie dorsale du châssis comprend une paroi transversale qui s'étend de la partie dorsale, de préférence de la paroi de la partie dorsale lorsqu'elle est présente, vers la partie frontale, ledit moyen de coulissement dont est munie la partie dorsale étant disposé sur une face supérieure de la paroi transversale (non illustré).

Le dispositif de renestage 8 comprend également un bloc peigne 25 muni d'une première partie latérale 26 et d'une deuxième partie latérale 27 s'étendant entre une base de bloc peigne et un ciel de bloc peigne 28. Le bloc peigne 25 comprend, entre ladite première partie latérale 26 et ladite deuxième partie latérale 27, n -1 dents de séparation 29 juxtaposées, et n espaces interdentaires 30, lesdites dents de séparation 29 et lesdits espaces interdentaires 30 étant orientés de manière sensiblement transversale par rapport à la direction d'alimentation A. Le bloc peigne 25 est disposé de telle façon que le ciel du bloc peigne 28 soit disposé à une hauteur mesurée à partir de la base dudit châssis h₂, chacun des n espaces interdentaires 30 étant agencé pour accueillir x seringues.

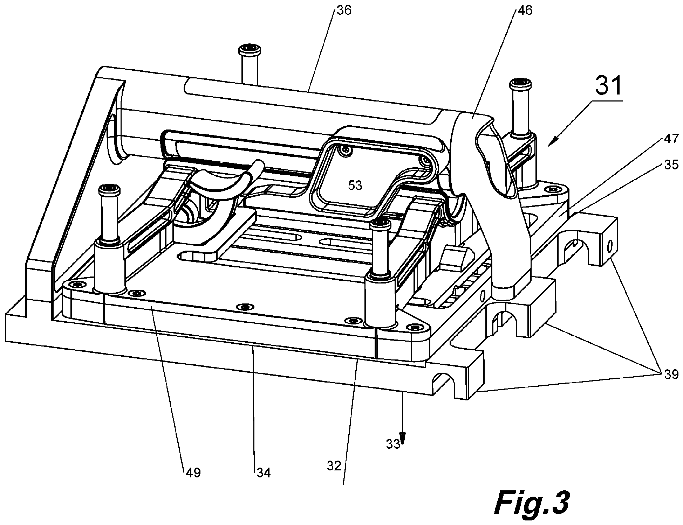

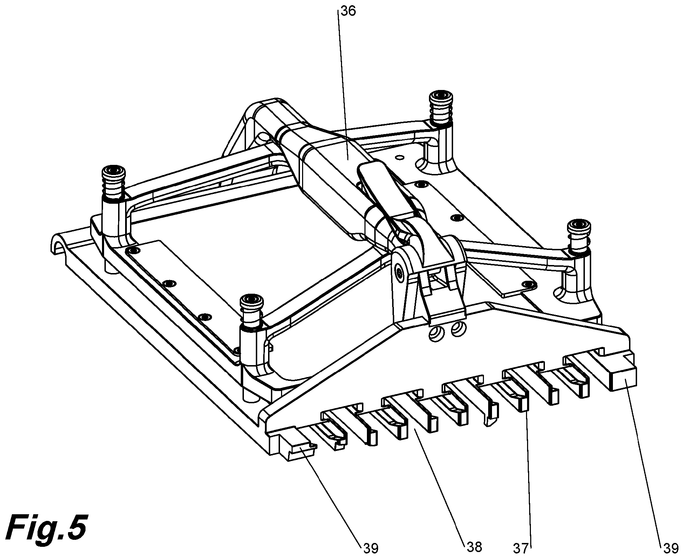

Le dispositif de renestage 8 comprend également un peigne mobile 31, comme on peut le voir aux figures 3, 4, 5 et 6. Le peigne mobile 31 est muni d'un ciel 32 de peigne mobile 31 et d'une base 33 de peigne mobile 31 de laquelle s'étendent une première partie latérale 34 et une deuxième partie latérale 35 et d'une poignée 36. Le peigne mobile 31 comprend entre ladite première partie latérale 34 et ladite deuxième partie latérale 35, x-1 dents de séparation 37 et x espaces interdentaires 38. Comme on peut le voir à la figure 6, les dents de séparation 37 et les espaces interdentaires 38 sont orientés de manière sensiblement parallèle à la direction d'alimentation A. Le peigne mobile comprend par ailleurs des moyens de coulissement mutuel 39 aux moyens de coulissements 17 de la partie dorsale 10 et de la partie frontale 16 dudit châssis 9.

Le peigne mobile 31 présente une position de déplacement selon laquelle il coulisse en appui sur ladite partie dorsale 10 et ladite partie frontale 16 dudit châssis qui lui permet de se déplacer transversalement par rapport à ladite direction d'alimentation A dans la direction du peigne bloc 25. La base de peigne mobile 31 est positionnée à une hauteur mesurée à partir de la base dudit châssis h₃ supérieure à h₂, lesdits espaces interdentaires étant ainsi agencés pour loger n seringues provenant dudit convoyeur de transport 15.

Comme on peut le voir à la figure 6, le dispositif de renestage 8 comprend un châssis 9 qui est placé en bout de convoyeur en ligne 15 dans lequel une partie dorsale 10 comprend une entrée 13 au travers de laquelle passent les seringues issues du convoyeur 15. Une butée 53 est présente. Elle s'étend de la base 12 dudit châssis 9 vers le haut et est agencée pour retenir les seringues en provenance de ladite ouverture 13, lorsque ledit peigne mobile 31 n'est pas en position de déplacement.

Les n espaces interdentaires 30 du bloc peigne 26 sont prévus pour accueillir chacun une série de x seringues. Ils présentent ainsi chacun une longueur suffisante pour accueillir ladite série. En d'autres mots, la longueur de chaque espace interdentaire 30 est supérieure au diamètre des (corps de) seringues multiplié par le nombre de seringues, soit x.

Les dents de séparation 29 du bloc peigne 26 permettent ainsi de séparer les séries de x seringues contenues dans les espaces interdentaires 30 les unes des autres de telle façon que la distance qui sépare les séries de x seringues logées dans les espaces interdentaires 30 soit sensiblement la même que celle qui sépare les séries de x seringues logées dans un nest 1.

Comme indiqué précédemment, les x espaces interdentaires 38 du peigne mobile 31 sont prévus pour accueillir chacun une série de n seringues. Ils présentent ainsi chacun une longueur suffisante pour accueillir ladite série. En d'autres mots, la longueur de chaque espace interdentaire 38 est supérieure au diamètre des (corps de) seringues multiplié par le nombre de seringues, soit n.

Les moyens de coulissement mutuels 39 du peigne mobile 31 sont engagés dans ceux du châssis 9, présents sur la partie dorsale 10 et sur la partie frontale 16. Ils permettent un déplacement du peigne mobile 31 perpendiculairement à la direction d'alimentation A de seringues. Le peigne mobile 31 peut ainsi se déplacer selon au moins x déplacements consécutifs. La position de déplacement du peigne mobile 31 est celle où les moyens de coulissement mutuels 39 du peigne mobile 31 sont engagés dans les moyens de coulissement 17 du châssis 9. Lors du premier déplacement du peigne mobile 31, le premier espace interdentaire 38 fait face à l'ouverture 13 de la partie dorsale 10 pour accueillir n seringues dans celui-ci. Ensuite, il est déplacé en direction du peigne bloc 26 et le deuxième espace interdentaire 38 fait face à l'ouverture 13 de la partie dorsale 10 pour également accueillir n seringues dans celui-ci. Cette opération est reproduite jusqu'à avoir le xième espace interdentaire 38 qui fait face à l'ouverture 13 de la partie dorsale 10 pour également accueillir n seringues dans celui-ci. Une fois le dernier (xième) espace interdentaire 38 rempli des n seringues, le peigne mobile 31 est encore déplacé en direction dudit peigne bloc 25.

Plus particulièrement, ledit peigne mobile 31 est agencé pour se déplacer selon un déplacement transversal par rapport à ladite direction d'alimentation A entre une première position p₁ et une dernière position p, en passant par x position successives, p₂, p₃, ...pₓ, ladite première position étant une position où le premier des x espaces interdentaire 38 fait face à ladite entrée 13 de la partie dorsale 10 dudit châssis 9, ladite dernière position p étant une position où chacun des x espaces interdentaire 38 a fait face à ladite entrée 13 de ladite partie dorsale 10 dudit châssis 9 et où les espaces interdentaires x contiennent les seringues de substance pharmaceutique ou cosmétique, ledit peigne mobile 31 étant alors positionné dans ladite dernière position de telle manière qu'il recouvre au moins partiellement ledit bloc peigne 25.

Cette manière de fonctionner est mise en oeuvre, dans la forme de réalisation illustrée à la figure 6, par la présence sur le moyen de coulissement 17 de creux 54 et sur le peigne mobile 31, d'un système de billes sur ressort ou de saillies dans les moyens de coulissement mutuels 39, lesdits creux 54 étant agencées pour recevoir lesdites billes sur ressort ou les dites saillies dudit peigne mobile 31.

Puisque la base 33 du peigne mobile 31 est positionnée à une hauteur h₃ mesurée à partir de la base 16 dudit châssis 9 qui est supérieure à h₂ (la hauteur h₂ étant la hauteur à laquelle culmine le ciel 28 du bloc peigne 25), lorsque le peigne mobile 31 se déplace en coulissant en direction du peigne bloc 26, il recouvre le peigne bloc 26 et les n seringues logées dans chacun des espaces interdentaires 38 du peigne mobile 31 sont engagées dans les n espaces interdentaires 30 du bloc peigne 25. Ainsi les x rangées de n seringues logées dans les x espaces interdentaires 38 du peigne mobile 31 sont logées dans les n colonnes (formées par les espaces interdentaires 30) du bloc peigne 25, au fur et à mesure du déplacement du peigne mobile 31 jusqu'à ce que chaque espace interdentaire 30 du bloc peigne 25 contienne une série de x seringues.

De cette manière, en utilisant le dispositif selon la présente invention, l'opérateur peut alors saisir le peigne mobile 31 qui repose sur les moyens de coulissement 17 dudit châssis 9 dans lequel les seringues pendent entre les dents 37, dans les espaces interdentaires 38 selon une matrice de n seringues sur x seringues correspondant aux orifices du nest 1 de n sur x seringues, pour effectuer la remise en nest 1 en le soulevant et en introduisant les seringues dans le nest 1.

Lorsque les seringues 2 pendent entre les dents 37, dans les espaces interdentaires 38, elles ont leurs collerettes qui reposent sur le ciel 32 du peigne mobile 31. Plus particulièrement une seringue 2 contenue dans un espace interdentaire 38 a sa collerette qui repose sur la face supérieure des dents 37 adjacentes à cet espace interdentaire 38.

Dans le peigne mobile 31, une série de n seringues est contenue dans chacun des x espaces interdentaires 38 et chaque série de n seringues 2 est espacée de la série suivante d'une distance correspondant à la fois à la largeur des dents de séparation 37 du peigne mobile et à la distance séparant chaque série de n seringues 2 dans le nest 1. Une fois le peigne mobile 31 recouvrant le bloc peigne 25, chaque seringue 2 de la série de n seringues 2 est introduite dans un espace interdentaire 30 du bloc peigne 25, opération durant laquelle elle est progressivement écartée de la suivante par les dents 29 du bloc peigne 25. Lorsque les x séries de n seringues 2 du peigne mobile 31 ont été introduites dans les n espaces interdentaires 30 du bloc peigne 25, chaque série des n séries de x seringues 2 est espacée de l'autre d'une distance correspondant à la fois à la largeur des dents de séparation 29 du bloc peigne 25 et à la distance séparant chaque série de x seringues dans le nest 1.

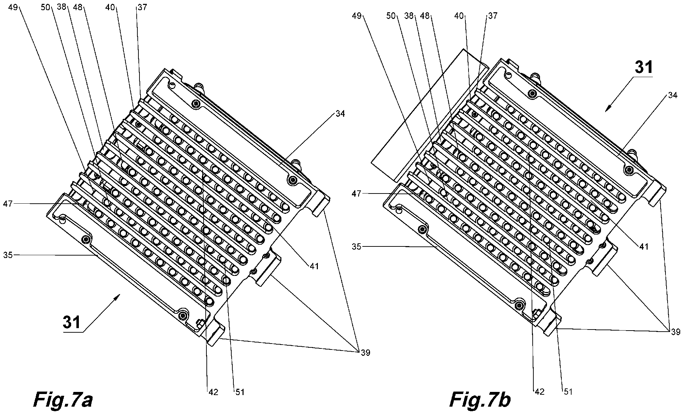

Comme on peut le voir plus particulièrement sur la figure 7, les dents 37 dudit peigne mobile 31 présentent chacune une extrémité libre 40 et une extrémité liée 41 entre lesquelles s'étend un corps 42 de dent 37, chaque extrémité libre 40 des dents 37 du peigne mobile 31 présentant une largeur s'amenuisant du corps 42 de dent 37 vers l'arête distale de ladite extrémité libre 40.

En faisant maintenant référence à la figure 6, les dents 29 dudit bloc peigne 25 présentent chacune une extrémité libre 43 et une extrémité liée 44 entre lesquelles s'étend un corps 45 de dent 29. Chaque extrémité libre 43 des dents 29 présentent une largeur s'amenuisant du corps 45 de dent 29 vers l'arête distale de ladite extrémité libre 43.

Ainsi, chaque corps 45 de dent 29 présente une largeur au niveau de l'extrémité libre 43 inférieure à une largeur au niveau de l'extrémité liée 44 du bloc peigne 25, de manière à permettre d'écarter les seringues les unes des autres.

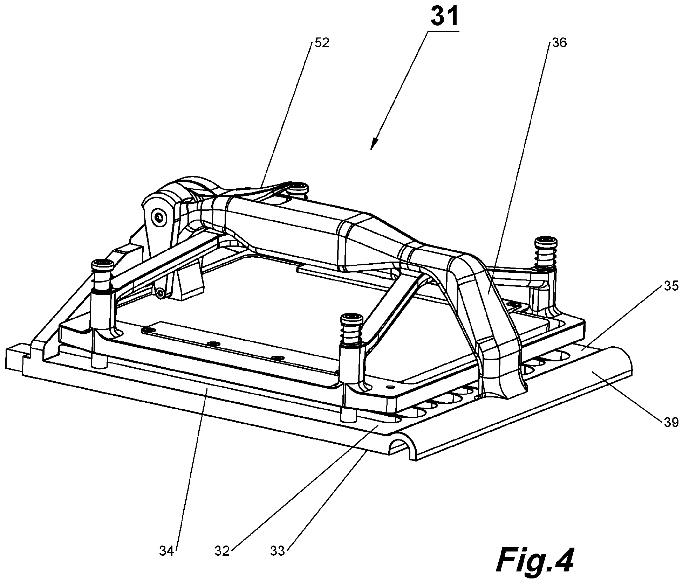

Comme illustré en détails aux figures 3 et 4, ledit peigne mobile 31 comprend une poignée 36, disposée au-dessus du ciel 32 de peigne mobile 31. La poignée 36 est reliée par des moyens de connexion 46 à l'un des éléments parmi le ciel 32 de peigne mobile 31, ladite première partie latérale 34 et ladite deuxième partie latérale 35 ou lesdits moyens de coulissement mutuels 39.

Les figures 7a et 7b illustrent le peigne mobile avec des moyens de centrage non activés et des moyens de centrage activés. Le peigne mobile 31 comprend en pratique des espaces interdentaires 38 pairs agencés pour loger chacun une série de n seringues et former des rangées paires de n seringues. Le peigne mobile 31 comprend aussi des espaces interdentaires 38 impairs agencés pour loger chacun une série de n seringues et former des rangées impaires de n seringues. Le peigne mobile 31 comprend des moyens de centrage agencés pour décaler les rangées paires de n seringues des rangées impaires de n seringues.

Une forme de réalisation des moyens de centrage est décrite ci-dessous, sans toutefois y être limité. Dans la description ci-dessous, il est fait référence à la figure 3 et aux figures 7a et 7b.

Dans la forme de réalisation illustrée, les moyens de centrage comprennent une première plaque 47 positionnée au-dessus des dents 37 du peigne mobile 31 en position de déplacement. Des orifices longitudinaux 48 sont présents dans la première plaque 47 et ils sont orientés dans une direction parallèle aux dents 37 du peigne mobile 31. Les orifices longitudinaux 48 surplombent ici les espaces interdentaires 38 pairs. Les moyens de centrage comprennent aussi une deuxième plaque 49 positionnée au-dessus de ladite première plaque 47 lorsque le peigne mobile 31 est en position de déplacement. La deuxième plaque comprend des saillies longitudinales 50 selon une direction parallèle aux dents 37 du peigne mobile 31. Les saillies longitudinales 50 sont prévues pour passer au travers desdits orifices longitudinaux 48 de la première plaque 47 et présentent une longueur inférieure à la longueur desdits orifices longitudinaux 48. Des éléments de reliefs 51 sont présents sous lesdites saillies 50, plus particulièrement n éléments de reliefs 51 s'étendant de ladite deuxième plaque 49 vers le bas lorsque le peigne mobile 31 est en position de déplacement. Les éléments de relief 51 surplombent les espaces interdentaires 38 impairs et ils sont prévus pour engager les orifices des corps des seringues présentes dans les espaces interdentaires 38 impairs du peigne mobile.

Les moyens de centrage comprennent en outre des moyens de commande 52 permettant un déplacement de ladite deuxième plaque 49 par rapport à ladite première plaque 47 lors duquel lesdites saillies longitudinales 50 de la deuxième plaque 49 effectuent un déplacement longitudinal dans les orifices longitudinaux 48 de la première plaque 47. Le déplacement longitudinal permet de déplacer les n seringues présentes dans les espaces interdentaires 38 pairs par rapport aux n seringues présentes dans les espaces interdentaires 38 impairs dudit peigne mobile 31.

Il est bien entendu que la présente invention n'est en aucune façon limitée aux formes de réalisations décrites ci-dessus et que bien des modifications peuvent y être apportées sans sortir du cadre des revendications annexées.

## Revendications

1. Dispositif de remise en nest (8) d'au moins n sur x (n multiplié par x) seringues (2) de substance pharmaceutique ou cosmétique comprenant :
- - un châssis (9) sensiblement parallélépipédique comprenant une partie dorsale (10) agencée pour être reliée à un convoyeur de transport (15) de seringues (2) de substance pharmaceutique ou cosmétique selon une direction d'alimentation (A) en au moins une file, ladite partie dorsale (10) s'étendant vers le haut à partir d'une base (12) du châssis (9) et comprenant une entrée (13) agencée pour permettre le passage de seringues (2) en provenance du convoyeur de transport (15) dans le châssis (9), une partie frontale (16) , s'étendant également vers le haut à partir de ladite base (12) du châssis et faisant face à ladite partie dorsale (10), la partie dorsale (10) et la partie frontale (16) étant munies de moyens de coulissement (17) disposé à une hauteur h1 mesurée à partir de ladite base (12) dudit châssis,
- - un bloc peigne (25) muni d'une première partie latérale (26) et d'une deuxième partie latérale (27) s'étendant entre une base de bloc et un ciel (28) de bloc peigne (25), et comprenant, entre ladite première partie latérale (26) et ladite deuxième partie latérale (27), n -1 dents de séparation (29) juxtaposées, et n espaces interdentaires (30), les dites dents de séparation (29) et lesdits espaces interdentaires (30) étant orientés de manière sensiblement transversale par rapport à la direction d'alimentation (A), et disposé de telle façon que le ciel (28) du bloc peigne (25) soit disposé à une hauteur h2 mesurée à partir de la base (12) dudit châssis (9), chacun des n espaces interdentaires (30) étant agencé pour accueillir x seringues (2),
- - un peigne mobile (31) muni d'un ciel (32) de peigne mobile (31) et d'une base (33) de peigne mobile (31) de laquelle s'étendent une première partie latérale (34) et une deuxième partie latérale (35) et d'une poignée (36), comprenant, entre ladite première partie latérale (34) et ladite deuxième partie latérale (35), x-1 dents de séparation (37) et x espaces interdentaires (38), lesdites dents de séparation (37) et lesdits espaces interdentaires (38) étant orientés de manière sensiblement parallèle à la direction d'alimentation (A) et comprenant des moyens de coulissement mutuels (39) aux moyens de coulissements (17) de la partie dorsale (10) et de la partie frontale (16) dudit châssis (9), ledit peigne mobile (31) présentant une position de déplacement où il est agencé pour coulisser en appui sur ladite partie dorsale (10) et ladite partie frontale (16) dudit châssis (9) et se déplacer transversalement par rapport à ladite direction d'alimentation (A) dans la direction du peigne bloc (25) et où ladite base (33) de peigne mobile (31) est positionnée à une hauteur h3 mesurée à partir de la base (12) dudit châssis (9), supérieure à h2, lesdits espaces interdentaires (38) étant agencés pour loger n seringues (2) provenant dudit convoyeur de transport (15).

2. Dispositif de remise en nest (8) d'au moins n sur x seringues (2) de substance pharmaceutique ou cosmétique selon la revendication 1, dans lequel ledit peigne mobile (31) est agencé pour se déplacer selon un déplacement transversal par rapport à ladite direction d'alimentation entre une première position p₁ et une dernière position p, en passant par x position successives, p₂, p₃, ...pₓ, ladite première position étant une position où le premier des x espaces interdentaire (38) fait face à ladite entrée (13) de la partie dorsale (10) dudit châssis (9), ladite dernière position p étant une position où chacun des x espaces interdentaire (38) a fait face à ladite entrée (13) de ladite partie dorsale (10) dudit châssis (9) et où les espaces interdentaires x (38) contiennent les seringues (2) de substance pharmaceutique ou cosmétique, ledit peigne mobile (31) étant alors positionné dans ladite dernière position de telle manière qu'il recouvre au moins partiellement ledit bloc peigne (25).

3. Dispositif de remise en nest (8) d'au moins n sur x seringues (2) de substance pharmaceutique ou cosmétique selon la revendication 1 ou la revendication 2, dans lequel ledit peigne mobile (31) comprend un poignée (36), disposée au-dessus du ciel de peigne (32) mobile, reliée par des moyens de connexion (46) à l'un des éléments parmi le ciel (32) de peigne mobile (31), ladite première partie latérale (34) et ladite deuxième partie latérale (35) ou lesdits moyens de coulissement mutuels (39).

4. Dispositif de remise en nest (8) d'au moins n sur x seringues (2) de substance pharmaceutique ou cosmétique selon l'une des revendications précédentes, dans lequel ledit peigne mobile (31) comprend des espaces interdentaires (38) pairs agencés pour loger chacun une série de n seringues (2) et former des rangées paires de n seringues (2) et des espaces interdentaires (38) impairs agencés pour loger chacun une série de n seringues (2) et former des rangées impaires de n seringues (2), et comprend des moyens de centrage (47) agencés pour décaler les rangées paires de n seringues (2) des rangées impaires de n seringues (2).

5. Dispositif de remise en nest (8) d'au moins n sur x seringues (2) de substance pharmaceutique ou cosmétique selon la revendication 4, dans lequel les moyens de centrage (47) comprennent
- une première plaque dans laquelle sont présents des orifices longitudinaux (48) orientés dans une direction parallèle aux dents (37) du peigne mobile (31), lesdits orifices longitudinaux (48) surplombant les espaces interdentaires (38) pairs, ladite première plaque étant positionnée au-dessus des dents (37) du peigne mobile (31) lorsque celui-ci est en position de déplacement et,
- une deuxième plaque positionnée (49) au-dessus de ladite première plaque lorsque le peigne mobile (31) est en position de déplacement et comprenant des saillies longitudinales (50) selon une direction parallèle aux dents (37) du peigne mobile (31), lesdites saillies longitudinales (50) étant agencées pour passer au travers desdits orifices (48) longitudinaux de la première plaque et de longueur inférieure à la longueur desdits orifices longitudinaux (48) et comprenant sous lesdites saillies (50), des éléments de reliefs (51) plus particulièrement n éléments de reliefs (51) s'étendant de ladite deuxième plaque (49) vers le bas, lesdits éléments de relief (51) surplombant les espaces interdentaires (38) impairs et étant agencés pour engager les orifices des corps des seringues (2) présentes dans les espaces interdentaires (38) impairs,
- des moyens de commande (52) permettant un déplacement de ladite deuxième plaque (49) par rapport à ladite première plaque lors duquel lesdites saillies longitudinales (50) de la deuxième plaque (49) effectuent un déplacement longitudinal dans les orifices longitudinaux (48) de la première plaque, ledit déplacement longitudinal permettant de déplacer les n seringues (2) présentes dans les espaces interdentaires (38) pairs par rapport aux n seringues (2) présentes dans les espaces interdentaires (38) impairs dudit peigne mobile (31).

6. Dispositif de remise en nest (8) d'au moins n sur x seringues (2) de substance pharmaceutique ou cosmétique selon la revendication 4, dans lequel les moyens de centrage (47) comprennent
- une première plaque dans laquelle sont présents des orifices longitudinaux (48) orientés dans une direction parallèle aux dents (37) du peigne mobile (31), lesdits orifices longitudinaux (48) surplombant les espaces interdentaires (38) impairs, ladite première plaque étant positionnée au-dessus des dents (37) du peigne mobile (31) lorsque celui-ci est en position de déplacement et,
- une deuxième plaque (49) positionnée au-dessus de ladite première plaque lorsque le peigne mobile (31) est en position de déplacement et comprenant des saillies longitudinales (50) selon une direction parallèle aux dents (37) du peigne mobile (31), lesdites saillies longitudinales (50) étant agencées pour passer au travers desdits orifices longitudinaux (48) de la première plaque et de longueur inférieure à la longueur desdits orifices longitudinaux (48) et comprenant sous lesdites saillies (50) , des éléments de reliefs (51), plus particulièrement n éléments de reliefs (51) s'étendant de ladite deuxième plaque (49) vers le bas, lesdits éléments de relief (51) surplombant les espaces interdentaires (38) pairs et étant agencés pour engager les les orifices des corps des seringues (2) présentes dans les espaces interdentaires (38) pairs,
- des moyens de commande (52) permettant un déplacement de ladite deuxième plaque (49) par rapport à ladite première plaque lors duquel lesdites saillies longitudinales (50) de la deuxième plaque effectuent un déplacement longitudinal dans les orifices longitudinaux (48) de la première plaque, ledit déplacement longitudinal permettant de déplacer les n seringues (2) présentes dans les espaces interdentaires (38) impairs par rapport aux n seringues (2) présentes dans les espaces interdentaires (38) pairs dudit peigne mobile (31) .

7. Dispositif de remise en nest (8) d'au moins n sur x seringues (2) de substance pharmaceutique ou cosmétique selon l'une quelconque des revendications précédentes, dans lequel ladite entrée (13) de la partie dorsale (10) est une ouverture en forme de U, dont l'ouverture est vers le haut, et d'une largeur l₁, formée dans une paroi (23) de la partie dorsale (10), dont les côtés (18, 19) forment à proximité de ladite ouverture une zone d'appui pour permettre à des collerettes de seringues (2) de reposer, ladite largeur l₁ étant supérieure au diamètre de corps des seringues (2).

8. Dispositif de remise en nest (8) d'au moins n sur x seringues (2) de substance pharmaceutique ou cosmétique selon l'une quelconque des revendications précédentes, dans lequel ladite partie dorsale (10) du châssis (9) comprend une paroi transversale qui s'étend de la partie dorsale (10), de préférence de la paroi de la partie dorsale (10) lorsqu'elle est présente, vers la partie frontale (16), ledit moyen de coulissement (17) dont est munie la partie dorsale étant disposé sur une face supérieure de la paroi transversale.

9. Dispositif de remise en nest (8) d'au moins n sur x seringues (2) de substance pharmaceutique ou cosmétique selon la revendication 7 ou la revendication 8, lorsqu'elle dépend de la revendication 7, dans lequel une paroi supérieure (21) est positionnée au-dessus de la paroi de la partie dorsale (10), ladite paroi supérieure (21) comportant une ouverture en forme de U (22), dont l'ouverture est orientée vers le bas, et présentant une largeur l₂ supérieure à la largeur l₁, l'ouverture (22) de la paroi de la partie dorsale et l'ouverture de la paroi supérieure formant ensemble une ouverture en forme de T, dont les épaulements forment ladite zone d'appui pour permettre à des collerettes de seringues de reposer.

10. Dispositif de remise en nest d'au moins n sur x seringues de substance pharmaceutique ou cosmétique selon l'une quelconque des revendications précédentes, dans lequel ladite entrée (13) de la partie dorsale (10) est munie de moyens de fermeture (20), agencés pour entraver le passage des seringues (2).

11. Dispositif de remise en nest (8) d'au moins n sur x seringues (2) de substance pharmaceutique ou cosmétique selon l'une quelconque des précédentes, dans lequel la partie dorsale (10) comprend un rail de guidage muni de creux et le peigne mobile (31) comprend un système de billes sur ressort, lesdits creux étant agencées pour recevoir lesdites billes sur ressort dudit peigne mobile.

12. Dispositif de remise en nest (8) d'au moins n sur x seringues (2) de substance pharmaceutique ou cosmétique selon l'une quelconque des revendications précédentes, dans lequel les dents (37) dudit peigne mobile (31) présentent chacune une extrémité libre (40) et une extrémité liée (41) entre lesquelles s'étend un corps de dent (42), chaque extrémité libre (40) des dents (37) présentant une largeur s'amenuisant du corps de dent (42) vers l'arête distale de ladite extrémité libre (40).

13. Dispositif de remise en nest (8) d'au moins n sur x seringues (2) de substance pharmaceutique ou cosmétique selon l'une quelconque des revendications précédentes, dans lequel les dents (29) dudit bloc peigne (25) présentent chacune une extrémité libre (43) et une extrémité liée (44) entre lesquelles s'étend un corps de dent (45), chaque extrémité libre (43) des dents présentant une largeur s'amenuisant du corps de dent (45) vers l'arête distale de ladite extrémité libre (43).

14. Dispositif de remise en nest d'au moins n sur x seringues de substance pharmaceutique ou cosmétique selon la revendication 13, dans lequel chaque corps de dent (45) dudit bloc peigne (25) présente une largeur au niveau de l'extrémité libre (43) inférieure à une largeur au niveau de l'extrémité liée (44), de manière à permettre d'écarter progressivement les seringues (2) les unes des autres.

15. Dispositif de remise en nest d'au moins n sur x seringues de substance pharmaceutique ou cosmétique selon l'une quelconque des revendications précédentes, dans lequel une butée est présente, s'étendant de la base dudit châssis vers le haut, et agencée pour se déplacer dans la direction d'alimentation des seringues, en face de ladite ouverture de ladite partie dorsale, ladite butée étant agencée pour retenir les seringues en provenance de ladite ouverture, avant que ledit peigne mobile présente le premier espace interdentaire et après que le peigne mobile présente le dernier espace interdentaire à ladite ouverture de la partie dorsale.

16. Procédé de remise en nest d'au moins n surx seringues (2) de substance pharmaceutique ou cosmétique en provenance d'un convoyeur de transport (15) auquel est fixé un châssis (9) muni d'un bloc peigne (25) comprenant une première partie latérale (26) et une deuxième partie latérale (27) s'étendant entre une base de bloc et un ciel (28) de bloc peigne (25), et comprenant, entre ladite première partie latérale (26) et ladite deuxième partie latérale (27), n -1 dents de séparation (29) juxtaposées, et n espaces interdentaires (30) et d'un peigne mobile (31) muni d'un ciel (32) de peigne mobile (31) et d'une base (33) de peigne mobile (31) de laquelle s'étendent une première partie latérale (34) et une deuxième partie latérale (35) et d'une poignée (36), ledit peigne mobile (31) comprenant, entre ladite première partie latérale (34) et ladite deuxième partie latérale (35), x-1 dents de séparation (37) et x espaces interdentaires (38), comprenant :
- Une alimentation de seringues (2) selon une direction d'alimentation (A) en ligne dans un premier espace interdentaire (38) dudit peigne mobile (31), lors de laquelle la première partie latérale (34) dudit peigne mobile est positionné au-dessus dudit ciel (28) de bloc peigne (25), jusqu'à obtenir une première rangée de n seringues (2) dans ledit premier espace interdentaire (38) x₁ dudit peigne mobile (31),
- Une série de x-1 cycle comprenant chacun un déplacement du peigne mobile (31) selon une direction perpendiculaire à ladite direction d'alimentation (A) en direction dudit bloc peigne (25), chaque déplacement permettant d'aligner un xième espace interdentaire (38) en face de ladite ouverture (13) et d'engager chacune des n seringues (2) d'une rangée précédentes de n seringues (2) dans un espace interdentaire (30) des n espaces interdentaires (30) dudit bloc peigne (25), et une alimentation de n seringues (2) dans le xième espace interdentaire (38) jusqu'à obtenir une xième rangée de n seringue (2) dans ledit xième espace interdentaire (38) dudit peigne mobile (31),
- Un blocage de la position des seringues (2) de x séries de n seringues logées dans le peigne mobile (31) et écartées les unes des autres par ledit bloc peigne (25) pour former une position de transfert desdites seringues (2) logées dans ledit peigne mobile (31),
- Un levage dudit peigne mobile (31) de manière à retirer lesdites seringues (2) bloquées en position de transfert dudit bloc peigne (25),
- Un déplacement dudit peigne mobile (31) jusqu'à le positionner au-dessus d'un nest (1) à seringues (2) de n sur x, de telle manière que lesdites seringues (2) logées dans ledit peigne mobile (31) soient placées au-dessus des orifices correspondants dans un nest (1) à seringues,
- Un engagement partiel desdites seringues (2) dans lesdits orifices correspondant dudit nest (1),
- Un retrait dudit peigne mobile (31) par déplacement sensiblement horizontal, permettant de libérer lesdites seringues (2) logées dans ledit peigne mobile (31) et partiellement dans lesdits orifices du nest (1) et leur descente dans l'orifice correspondant une fois libérée dudit peigne mobile (31) pour obtenir une mise en nest (1) des seringues (2).

17. Procédé de remise en nest d'au moins n surx seringues (2) de substance pharmaceutique ou cosmétique en provenance d'un convoyeur de transport (15), comprenant préalablement audit blocage de la position des seringues (2) logées dans le peigne mobile (31), un déplacement des seringues (2) des rangées paires par rapport aux seringues (2) des rangées impaires de manière à former des rangées de seringues en quinconce les unes par rapport aux autres.
